# EUROPEAN PATENT SPECIFICATION

(11) **EP 2 931 302 B1**
(45) Date of publication and mention of the grant of the patent: **25.11.2020**
(21) Application number: 12858356.4
(22) Date of filing: 14.12.2012
(51) Int. Cl.: A61K 38/39, C12N 5/078

(54) **DECELLULARIZED SMALL PARTICLE TISSUE**
DEZELLULARISIERTES FEINTEILIGES GEWEBE
TISSU À PETITES PARTICULES DÉCELLULARISÉ

(43) Date of publication of application: 21.10.2015
(73) Proprietor: Burke, Edmund, Jersey City, NJ 07302 (US)
(72) Inventor: Burke, Edmund, Jersey City, NJ 07302 (US)
(74) Representative: Teuten, Andrew John
(86) International application number: PCT/US2012/000577
(87) International publication number: WO 2013/089808

(56) References cited:
- WO-A2-2006/135843
- WO-A2-2007/009036
- US-A1- 2002 028 278
- US-A1- 2002 102 727
- US-A1- 2002 115 208
- US-A1- 2006 166 361
- US-A1- 2007 020 225
- US-A1- 2010 028 396

## Description

### CROSS REFERENCE TO RELATED APPLICATIONS

This Application claims the benefit of United States Provisional Application Number 61/630,561 filed December 14, 2011.

### FIELD OF THE INVENTION

The technical field to which this invention relates is the field of producing and decellularizing tissue. The art abounds with numerous techniques for producing decellularized tissues which are widely useable in such areas as tissue repair, tissue regeneration, wound repair, cell growth media or substrates, filling skin defects and voids, tissue implantation, skin grafting and regrowth, organ repair and organ transplantation and other similar areas.

### BACKGROUND OF INVENTION

The numerous and varied techniques, methods and processes for producing decellularized tissue usually start with tissue from a variety of sources including organs and epithelial tissue and various other sources depending on the needs for the ultimate use of the decellularized tissue. The repair or treatment of various body tissues, such as skin, organs, and the like, has been accomplished using collagen compositions, including tissue membranes comprising collagen, e.g., amniotic membrane, pericardium, dura mater, and the like. A need exists, however, for additional, more versatile compositions that can be used in medical applications in addition, to, or in place of, membranes.

A major difficulty with most of the wide variety of processes and techniques that have been used to decellularize tissue, has been achieving the removal of the cells from the tissue while maintaining in the tissue, beneficial proteins from the starting materials which may be needed in the subsequent use of the decellularized tissue in its medical environment. Similarly, while it is desirable, and in some cases necessary, to retain beneficial proteins, it is also undesirable to retain debris and cellular materials resulting from the decellularizing process since they may cause deleterious effects upon subsequent implantation into the body. Undesirable products which come to mind in this respect are whole cells, nucleic acids, endotoxins and other degradation products.

Decellularized tissues are generally produced in a variety of physical forms, usually as large pieces on which cells may grow. Nevertheless, it has been difficult to produce a small particle tissue while avoiding the difficulties expressed above. Advantages of small particle decellularized tissue include but are not limited to the ability to mold and dry it into a variety of shapes, or to making it injectible, making it filter sterilizable and making it more advantageous for cells to utilize in culture conditions.

The decellularized tissue production techniques normally employed in the art utilize decellularizing agents which are usually oxidizing materials such as hydrogen peroxide, with other ingredients such as a detergent, ethanol, or some other oxidizing material, such as peracetic acid, in the presence of such materials as ferrous salts, copper salts, enzymes and the like. Other methods may be employed using combinations of acids, bases and chelating agents to remove cellular remnants from the starting material. These methods often yield decellularized tissues which are usually unsatisfactory in one way or another, however.

Other techniques involve methods which use harsh materials such as sodium hydroxide or hydrochloric acid under harsh conditions such as extremes in pH, without yielding generally suitable results. Most of these methods are generally unwieldy requiring much manipulation in batch type processes and many of them, especially non-oxidizing acid extractions are not effective decellularizing agents and beneficial proteins remain unextracted to an unsuitable extent. In this regard, the distinction between a mere extraction process and the present inventive decellularizing, particle reduction process should be clear.

In short, it is felt that the present state of the prior art, while sufficient in some cases to remove some cellular materials, leaves much to be desired in terms of the quality of the decllularized tissue, especially in the loss of beneficial proteins remaining after the treatment with the decellularizing agents. The present invention intends to provide a novel process which balances the beneficial proteins remaining in the decellularized, small particle tissue with the removal of materials that are undesirable and yet yield a novel, decellularized, small particle tissue which can be widely used in a variety of indications.

### SUMMARY OF THE INVENTION

The present invention is operable with a variety of starting tissues, such as animal or human placenta, skin, liver, kidney, spleen and the like, including blood itself. Mainly, we utilize what we term herein as "blood-laden" tissue. By "blood laden" is meant tissue which contains a significant amount of blood after removal of the tissue from the body. A preferred tissue of major use in this invention is the highly vascularized placenta and other highly vascularized organs.

In one aspect of the invention, tissue which is not blood-laden or which has for some reason lost its blood, may be rendered blood-laden by supplying exogenous blood thereto and be useful in the invention. We have found that amounts of blood in the tissue relative to the volume of cells in the tissue can be varied over a wide range to yield suitable results. Indeed, blood itself would be a suitable starting material.

In its general applicability, and according to one broad embodiment, the present invention does not rely specifically on the particular decellularizing agent that is used to treat the tissue. That is, virtually any decellularizing agent or agents that work satisfactorily to produce decellularized tissue convenient to the uses of the practitioner, would be suitable. In this broad embodiment, and in fact, in other embodiments, a critical step lies in a treatment step following the decellularization and size reduction step, which comprises a sequential particle size separation and recycling system. In such decellularization techniques, where blood-laden materials are not usually employed, the invention is effective via the sequential particle size separation and recycling technique described herein. Particle size separation may be accomplished by any means known to those skilled in the art, including e.g., centrifugation and filtration.

Thus, the tissue decellularizing techniques utilized in the broad embodiment of present invention may be any of the techniques that are normally used in the art. Typically, the existing art tissue decellularizing techniques, comprising for example, systems containing hydrogen peroxide and ethanol, or hydrogen peroxide with peracetic acid or a detergent, or enzymes, or deoxycholic acid, have been used in the art in some circumstances. In the broad embodiment, following the step in which any decellularizing system may be employed, the present invention utilizes a novel particle size separation and recycling system.

In view of the nature of the reactants of the decellularization and particle size reduction process, and our novel recycling system of the unspent reagents used in the decellularization and particle size reduction step, we are able to use less manipulation of reagents and yet speed up the process of decellularization and particle size reduction. In that regard, it should be noted that the first treatment of tissue in the art whether it includes particle size reduction or not, generally does not consume all of the reagents that are applied to decellularizing the tissue, thus resulting in a very inefficient process. Indeed, when using, for example, a peroxide/peracetic acid mixture as used in the prior art there is sufficient peroxide and acid still remaining in the mixture after treatment to further decellularize tissue remaining in the batch. Yet, in conducting the technique, the art would be content to isolate the treated tissue and expect to have sufficiently purified tissue available to it. The actual result is to the contrary. The resulting decellularized tissue from the art techniques is usually not satisfactory and the process is extremely inefficient and expensive. We have discovered that by using particle size separating methods and parameters that sequentially separate smaller particle sizes, we are able to remove product from the system that is in a desired range of particle size and direct the final effluent back to any upstream stage of particle size separation or the decellularization and particle size reduction step.

### DETAILED DESCRIPTION OF THE INVENTION

The invention relates to the preparation of a novel, decellularized small particle tissue. Both the product and the process for making it are novel.

The decellularized small particle tissue of the invention can be injectible when the tissue is reduced to a certain particle size and dispersed in a suitable liquid although it may be used in a solid form as well. The term "small particle", as used herein, is meant to apply to extremely small size particulates of tissue or the tissue components. When present in a liquid vehicle, the novel product is injectible into various body locations. Used as a final small particulate form, it is also useable in many locations on the body and can be used as a void filler, for example. These aspects will be apparent when the description of preparation is considered.

The process of the invention starts with an appropriate blood-laden source of tissue to be decellularized. The term "blood-laden" is meant to be descriptive of any tissue which retains a residual amount of blood as part of the tissue starting material to be decellularized, bearing in mind that in some cases blood itself could be a starting tissue. Additionally, extraneous blood can be allowed to drip off, leaving tissue containing a suitable amount of residual blood. Thus, our decellularizing reaction can be said to be one which is carried out on appropriate tissue in the presence of an amount of blood effective in the decellularizing treatment, without regard to the actual amount of blood associated with the tissue. One skilled in the art will quickly detetermine an amount which leads to optimum results in carrying out the process on the particular tissue chosen by the user. In some cases, blood can be added to tissue deemed to be insufficient in the amount of blood present.

In the usual decellularizing process, the tissue is treated first with a decellurizing agent, then washed and dried to produce the final product. Many and various decellularizing agents have been used. In one broad aspect of the invention, any decellularizing agent may be employed, since it is believed that the benefits of the invention are more pronounced, at least in this one aspect, when the sequential recycling steps employed following the decellularizing step, as described below, are employed.

In this regard, the first step of the invention in the broadly described aspect is submitting tissue which has preferably been minced and homogenized, to any decellularizing technique, whether or not there is blood present in the tissue. Thus, we are here speaking of any tissue and any decellularizing technique in the first step of the broad aspect of the invention. Appropriate adjustment in the decellularizing agents will be made when insufficient blood remains in the tissue or additional blood is added if it is desired to employ the blood-laden embodiment. There results a partial removal of cellular components, and other debris in a process which tends to be incomplete and inefficient.

In our inventive process, the entire reaction product mixture obtained as described above, is passed through a particle size separation system comprised of a series of stages, at least more than one stage, designed to capture sequentially smaller particle sizes, the effect of which is to permit unreacted decellularized reagents and tissue to continue their decellularizing and particle size reduction effect while at the same time capturing only the smallest decellularized tissue product, depending on the parameters of the particle size separation system. The effluent (smaller size range of separated particles) from the particle size separation system or any stage thereof, is repeatedly recycled to either or both of the decellularizing and particle size reduction step, to any previous stage of the particle size separation system, to cause repeated decellularization and particle size reduction. The retentate (larger size range of separated particles) of the particle size separation can likewise be recycled upstream to any point in the process. This can be seen more effectively by reference to the flow diagram submitted herewith.

As noted above, in the broad concept of the invention, one may employ any suitable decellularizing agents followed by the repetitive recycling through any suitable size separation system particle size. A preferred embodiment of the invention, however, is one where the starting tissue is blood-laden tissue as aforementioned and the decellularizing agent is hydrogen peroxide (H₂O₂). A preferred size separation system is a plurality of filters, i.e., at least two filters, having sequentially smaller filter sizes. In general, the pore size of the filters of the filtering step range down, for example, from about 100 microns to 50 microns, to tenths of microns and most preferably, to 100 Kilo Dalton to 10 Kilo Dalton. At these sizes, the tissue is considered to be of small particle size within the meaning of the present invention. This is preferably accomplished in a plurality of filter stages of at least two filters stages of different pore sizes. The ultimate lowest size is preferably below 50 Kilo Dalton, but a suitable decellularized tissue product may be obtained at a final filter stage of about 100 Kilo Dalton.

A preferred tissue is blood-laden placenta. We have discovered that the use of the novel, repetitive, recycling system of the invention with the blood-laden tissue enables the use of hydrogen peroxide (H₂O₂) alone, i.e., without the added use of an adjunctive material such as peracetic acid, a detergent, copper salts e.g., copper chloride, or ferrous salts, e.g., ferrous sulfate, or other adjunctive decellularizing agents. One may, if desired, use any of such additional materials, but their presence is not necessary to achieve the results of the invention. The advantage of eliminating the need for additional decellularizing agents is at once apparent. Costs are reduced for one, and the process is much easier to automate if reactants need not have to be used and replenished. There is much less manipulation than in processes using more reactants, in that there are fewer residual materials that need to be washed out.

The temperature at which the decellularizing process of the invention is performed is suitably from about 2°C to room temperature or slightly above. Temperatures ranging from 5°C to about 30°C are suitable, but temperatures high enough to cause gel formation of the collagen structure of the tissue should be avoided. The pH of the process is suitably on the acid side, ranging from 2.5 to 7 and preferably 3 to 5, to produce suitable results.

After the process operations are completed, the product is isolated by any of a variety of techniques, including simple drying of the product at slightly elevated temperatures above room temperature, or lyophilizing of the product, or any such method. Raising the pH before or after lyophilization to from 6 to about 8, facilitates this procedure. The final product may be sterilized in various ways if desired. Preferably, the final product also is dried to a moisture content of less than 80%

Reference to the flow diagram will illustrate aspects of the invention. The flow diagram headed "Decellularized Small Particle Tissue Process Flow Using 2 Filters," gives a general schematic flow diagram of the various steps involved in the process of the invention to produce the small particle tissue. An important consideration of the invention is that the reaction scheme employs the decellularization and low pH treatment steps simultaneously or at approximately the same time. In the first step, the blood-laden tissue, which is usually stored frozen, is thawed and then homogenized along with effluent blood to reduce the larger sized tissue into smaller pieces. In this regard, tissue may be cut into pieces of, for example, 50 mm or lower to provide a conveniently handled medium.

Next, the pH is adjusted to, for example, approximately 3 to 6 and then the mixture further mixed, blended, or homogenized in a reaction vessel, and the pH preferably maintained between 4 and 6. The mixture is then treated with hydrogen peroxide and the product mixed for an appropriate time, usually two to twenty-four hours to decellularize and reduce the particle size of the tissue.

Thereafter, the reactor vessel mixture is passed through a first filter of 0.2 - 200 microns and the filtrate then passed through a 10 Kilo Dalton to 0.2 micron filter to produce more filtrate and retentate.

The filtrates from either or both of the first and second filters are recycled repetitively to the reaction vessel until from about 80% to most, if not all, of the solids are removed from the reaction vessel. In addition, the filtrate from the second filter may be recycled to the first at will and as desired. The ultimate product will be the retentate from the second or last filter.

With regard to the filtering and recycling steps, while the flow diagram shows two filters, the progression through the filter pore size range of 200 microns down to 0.2 microns may be achieved in more than 2 stages. Three or four filters are usually acceptable with the pore sizes decreasing from 200 microns or larger sizes down to 10 KD gradually from the first to the last filter. The product is then isolated using normal techniques, such as by air drying at elevated temperatures above room temperatures or preferably by lyophilizing as indicated below.

While the 200 micron level has been referred above, a larger pore size may be selected for the first filter to provide somewhat more facile processing. The lower end of the range may be any achievable, but for practical reasons, including ease of processing, the lower range pore size is conveniently from about 10 to 50 KD.

The novel compounds of the invention may be characterized as the product which is produced from any of the processes or its variations as set forth above and has the characteristics set forth below.

The decellularizing and particle size reduction process of the present invention is effective to produce a product which retains beneficial proteins from the starting tissue to a greater extent than has been achieved in the prior art processes.

The particle size separation system may be any system which effects a particle size reduction such as centrifugation, or filtering systems, for example, provided the size reduction and recycling steps as recited herein are used. The resulting biomaterial composition of the present invention is comprised of tissue that has been treated with a decellularizing agent and then passed through a particle size separation system comprised of a series of stages designed to capture sequentially smaller particle sizes. The effect of this is to permit unreacted decellularized reagents and tissue to continue their decellularizing and particle size reduction effect while at the same time removing as effluent only the smallest decellularized tissue product and soluble reaction media depending on the parameters of the particle size separation system or any stage thereof. The latter stage involves recycling to either or both of the decellularizing and particle size reduction steps to cause repeated decellularization and particle size reduction while retaining the desireable elements of the decellularized tissue in suitable amounts. The retentate (larger size range of separated particles) of any stage of the particle size separation system can likewise be recycled upstream to any point in the process.

As a result, we are able to obtain a novel product capable of being characterized as follows:

A biomaterial comprising tissue that has previously been treated with a decellularizing agent, in a process wherein said tissue has been treated in a particle size separation system capable of separating particle sizes in the range of from about 10 Kilo Daltons to about 200 microns and further comprises the following constituents presented below. The amount of the constituents may be expressed in at least two ways. We prefer to express them here as percent by weight on the basis of the starting biomaterial tissue as one measure and as a percent by weight of the constituent based on the total dry weight of the biomaterial product. Using these parameters, the constituents have the following concentrations:

As a percentage by weight of the dry starting biomaterial tissue concentration:
Collagen, at least 40% and preferably from 60% to 70%;
Elastin, at least 50% and preferably from 70% to 90;
Laminin, at least 10% and preferably from 12% to 21%;
Fibronectin, at least 30% and preferably from 40% to 60%;
ds DNA of less than 10% to 15% and preferably less than 5%; and

As a percentage of the dry weight of the final biomaterial product:
Collagen of at least 30% and preferably from 40% to 50%,
Elastin of at least 2% and preferably from 4% to 5%,
Laminin of at least 0.025% and preferably from 0.045% to 0.01%,
Fibronectin of at least 0.10% and preferably from 0.12% to 0.3%,
Glycosaminoglycan of at least 0.2% and preferably from 0.3% to 0.5%,
ds DNA less than 0.1%, and
Endotoxin less than 0.1% EU/mg.

The utility of the final product may be exemplified by:
- as a material to heal wounds, to culture cells, as a bone filler, to fill cartilage and tendon, a material to activate cells prior to application or injection, as a material to activate or emit a cellular response in vivo, construction of manmade scaffolding,
- further treatment to effect viral inactivation such as pH swings, detergent washing etc.,
- filling syringes for use in dermatological procedures,
- lyophilizing into sheets or sponges for wound coverings,
- spray drying particles for cell growth media,
- cross-linking to act as filters for plastic surgery, bone void fillers and the like, and
- preparing various shapes as above for multiple various uses in the medical arts.

While the invention has been described in detail and with reference to specific examples thereof, it will be apparent to one skilled in the art that various changes and modifications can be made therein without departing from the scope thereof.

As additional aspects of the invention, the following may be mentioned when filters are used, bag filters, and cross flow membrane filters may be employed, decellularization may be aided by employing sonication frequencies, starting material for blood laden tissue may be umbilical cord as well as placenta.

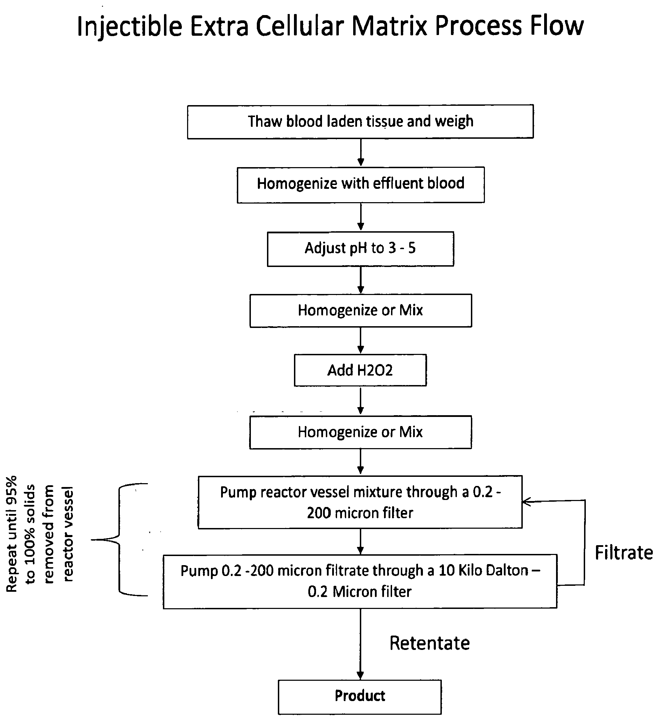

## Claims

1. A process for producing a decellularized small particle tissue which comprises the following steps:
(a) Selecting an appropriate tissue starting material from which a decellularized small particle tissue is desired to be prepared, wherein the tissue is blood-laden tissue;
(b) treating the blood-laden tissue with a decellularizing agent at an acid pH to remove at least a portion of the cellular material therefrom and to yield a product comprising a liquid component and a solid component,
(c) subjecting the liquid component and the solid component which is the product of step (b) to a particle separation system comprised of a plurality of filters, F₁ through Fₙ, wherein n may be 2, or an integer higher than 2, wherein the pore size of the filter F₁ is 0.2 to 200 microns, and the pore size of the filters Fₙ range from 100 microns to 50 microns or range from 100 Kilo Daltons to 10 Kilo Daltons, yielding filtrates and retentates,
(d) recycling either of said filtrates or said retentates or both, either separately or together, to any of steps b) or c) or both, at least one time,
(e) isolating a decellularized small particle tissue from any of steps b), c) or d);
wherein the pore size of the sequentially last stage filter is between 10 Kilo Daltons-50 Kilo Daltons and wherein the particle separation system is designed to capture sequentially smaller particle sizes.

2. The process according to claim 1 wherein the blood-laden tissue starting material is a blood-laden placenta or umbilical cord.

3. The process according to claim 1 or claim 2 wherein the decellularizing agent is an oxidizing agent.

4. The process according to claim 3 wherein the oxidizing agent is hydrogen peroxide.

5. The process according to any one of claims 1 to 4 wherein the plurality of filters in step c) is 2 filters.

6. The process according to any one of claims 1 to 5 wherein the pH of step b) is from 2-7.

7. The process according to any one of claims 1 to 6 wherein the filtrate from any filter that is not the sequentially last stage filter (smallest pore size) is passed to the next filter that has the sequentially smaller pore size; and
wherein the filtrate from the sequentially last stage (smallest pore size) is recycled to any
filter or to the decellularizing and size reduction step a) or both.

8. The process according to any one of claims 1 to 7, wherein prior to step e), the pH is adjusted to between 6 and 8.

9. The process according to any one of claims 1 to 8, wherein step e) includes lyophilizing the small particle tissue product.

## Patentansprüche

1. Verfahren zur Herstellung eines dezellularisierten feinteiligen Gewebes, das die folgenden Schritte umfasst:
(a) Auswählen eines geeigneten Gewebeausgangsmaterials, aus dem ein dezellularisiertes feinteiliges Gewebe hergestellt werden soll, wobei es sich bei dem Gewebe um blutbeladenes Gewebe handelt;
(b) Behandeln des blutbeladenen Gewebes mit einem Dezellularisierungsmittel bei einem sauren pH, so dass wenigstens ein Teil des zellulären Materials davon abgetrennt und ein Produkt erhalten wird, das eine flüssige und eine feste Komponente umfasst,
(c) In-Kontakt-Bringen der flüssigen und der festen Komponente, wobei es sich um das Produkt aus Schritt (b) handelt, mit einem Partikeltrennungssystem, das aus mehreren Filtern, F₁ bis Fₙ, besteht, wobei n gleich 2 oder eine ganze Zahl größer 2 sein kann, wobei die Porengröße des Filters F₁ 0,2 bis 200 Mikrometer beträgt und die Porengröße der Filter Fₙ im Bereich von 100 Mikrometer bis 50 Mikrometer oder im Bereich von 100 Kilodalton bis 10 Kilodalton liegt, wobei Filtrate und Retentate erhalten werden,
(d) Rückführen entweder der Filtrate oder der Retentate oder von beiden entweder getrennt oder zusammen wenigstens einmal zu Schritt b) oder bzw. und Schritt c),
(e) Isolieren eines dezellularisierten feinteiligen Gewebes aus einem der Schritte b), c) oder d);
wobei die Porengröße des Filters der im Ablauf letzten Stufe zwischen 10 Kilodalton-50 Kilodalton liegt und wobei das Partikeltrennungssystem zum Einfangen sequentiell kleinerer Partikelgrößen vorgesehen ist.

2. Verfahren nach Anspruch 1, wobei es sich bei dem blutbeladenen Gewebeausgangsmaterial um eine blutbeladene Plazenta oder Nabelschnur handelt.

3. Verfahren nach Anspruch 1 oder Anspruch 2, wobei es sich bei dem Dezellularisierungsmittel um ein Oxidationsmittel handelt.

4. Verfahren nach Anspruch 3, wobei es sich bei dem Oxidationsmittel um Wasserstoffperoxid handelt.

5. Verfahren nach einem der Ansprüche 1 bis 4, wobei es sich bei den mehreren Filtern in Schritt c) um 2 Filter handelt.

6. Verfahren nach einem der Ansprüche 1 bis 5, wobei der pH-Wert in Schritt b) 2-7 beträgt.

7. Verfahren nach einem der Ansprüche 1 bis 6, wobei das Filtrat von jedem Filter, bei dem es sich nicht um den Filter der im Ablauf letzten Stufe (kleinste Porengröße) handelt, zum nächsten Filter, der die nächstkleinere Porengröße aufweist, geleitet wird; und
wobei das Filtrat aus der im Ablauf letzten Stufe (kleinste Porengröße) zu einem beliebigen Filter oder zum Dezellularisierungs- und Verkleinerungsschritt a) oder beiden zurückgeführt wird.

8. Verfahren nach einem der Ansprüche 1 bis 7, wobei vor Schritt e) der pH auf einen Wert zwischen 6 und 8 eingestellt wird.

9. Verfahren nach einem der Ansprüche 1 bis 8, wobei Schritt e) Lyophilisieren des feinteiligen Gewebeprodukts beinhaltet.

## Revendications

1. Procédé de production d'un tissu de petites particules décellularisé qui comprend les étapes suivantes :
(a) sélection d'un matériau de départ de tissu approprié à partir duquel il est souhaité de préparer un tissu de petites particules décellularisé, dans lequel le tissu est un tissu chargé de sang ;
(b) traitement du tissu chargé de sang avec un agent de décellularisation à un pH acide pour retirer au moins une partie du matériau cellulaire de celui-ci et pour obtenir un produit comprenant un composant liquide et un composant solide,
(c) soumission du composant liquide et du composant solide qui sont le produit de l'étape (b) à un système de séparation de particules constitué d'une pluralité de filtres, F₁ à Fₙ, dans lequel n peut être 2, ou un entier supérieur à 2, dans lequel la taille de pore du filtre F₁ est de 0,2 à 200 microns, et la taille de pore des filtres Fₙ est dans la plage de 100 microns à 50 microns ou dans la plage de 100 kilodaltons à 10 kilodaltons, pour obtenir des filtrats et des rétentats,
(d) recyclage desdits filtrats ou desdits rétentats ou des deux, séparément ou conjointement, à l'une quelconque des étapes b) ou c) ou les deux, au moins une fois,
(e) isolement d'un tissu de petites particules décellularisé à partir de l'une quelconque des étapes b), c) ou d) ;
dans lequel la taille de pore du filtre séquentiellement de dernière étape est comprise entre 10 kilodaltons et 50 kilodaltons et dans lequel le système de séparation de particules est conçu pour capturer des tailles de particule séquentiellement plus petites.

2. Procédé selon la revendication 1 dans lequel le matériau de départ de tissu chargé de sang est un placenta ou un cordon ombilical chargé de sang.

3. Procédé selon la revendication 1 ou la revendication 2 dans lequel l'agent de décellularisation est un agent oxydant.

4. Procédé selon la revendication 3 dans lequel l'agent oxydant est le peroxyde d'hydrogène.

5. Procédé selon l'une quelconque des revendications 1 à 4 dans lequel la pluralité de filtres dans l'étape c) est 2 filtres.

6. Procédé selon l'une quelconque des revendications 1 à 5 dans lequel le pH de l'étape b) est de 2 à 7.

7. Procédé selon l'une quelconque des revendications 1 à 6 dans lequel le filtrat provenant d'un filtre quelconque qui n'est pas le filtre séquentiellement de dernière étape (plus petite taille de pore) est passé au filtre suivant qui a la taille de pore séquentiellement la plus petite ; et
dans lequel le filtrat de la dernière étape séquentiellement (plus petite taille de pore) est recyclé vers un filtre quelconque ou vers l'étape de décellularisation et de réduction de taille a) ou les deux.

8. Procédé selon l'une quelconque des revendications 1 à 7, dans lequel, avant l'étape e), le pH est ajusté entre 6 et 8.

9. Procédé selon l'une quelconque des revendications 1 à 8, dans lequel l'étape e) comprend la lyophilisation du produit de tissu à petites particules.
